# EUROPEAN PATENT APPLICATION

(11) **EP 4 752 130 A1**
(43) Date of publication of application: **03.06.2026**
(21) Application number: 24845231.0
(22) Date of filing: 12.06.2024
(51) Int. Cl.: C07C 69/145, A23L 2/56, A23L 27/20, A24B 15/30, A61K 8/37, A61Q 13/00, C11B 9/00

(54) **FRAGRANCE COMPOSITION**

(30) Priority: 25.07.2023 JP 2023120737
(71) Applicant: Takasago International Corporation, Tokyo 144-8721 (JP)
(72) Inventor: MURATA, Ariaki, Hiratsuka-shi, Kanagawa 254-0073 (JP); KURABE, Aki, Hiratsuka-shi, Kanagawa 254-0073 (JP); HARADA, Makoto, Hiratsuka-shi, Kanagawa 254-0073 (JP)
(74) Representative: Zimmermann & Partner Patentanwälte mbB
(86) International application number: PCT/JP2024/021337
(87) International publication number: WO 2025/022849

(57) **Abstract**

The purpose of the present invention is to provide a fragrance composition which is capable of enhancing citrus-like aroma and flavor. The present invention relates to a fragrance composition which contains (5E)-6,10-dimethyl-11-oxoundeca-5,9-dien-2-yl acetate.

## Description

### Technical Field

The present invention relates to a compound and a flavor/fragrance composition useful as flavor/fragrance ingredients for blending and the like.

### Background Art

In recent years, as consumer preferences have become more diverse, highly fruity and natural flavors and flavors distinctive from others have become strongly demanded as flavors and/or fragrances for use in foods and/or beverages and others. However, it is sometimes difficult to meet these demands with combinations of conventional flavor/fragrance ingredients, and novel flavor/fragrance materials are in high demand.

### Summary of Invention

The present invention is intended to provide a novel compound having a scent useful as a flavor/fragrance ingredient for blending or the like, and a flavor/fragrance composition comprising the compound.

As a result of synthesizing various compounds and examining their scents, the present inventors found that a compound having an aldehyde group and an ester structure at its terminals has an excellent scent.

Specifically, the present invention relates to the following [1] to [6].
[1] A compound represented by the following formula (1): 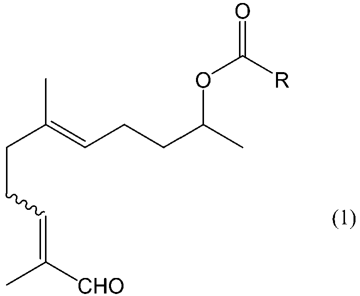 where R represents an alkyl group having 1 to 4 carbon atoms and a wavy line represents cis, trans, or a mixture of cis and trans.
[2] The compound according to the [1], wherein R is a methyl group.
[3] A flavor/fragrance composition comprising the compound according to the [1] or [2].
[4] The flavor/fragrance composition according to the [3], which is for a food and/or beverage.
[5] A food and/or beverage, an oral composition, a tobacco product, or a consumer product comprising the compound according to the [1] or [2], or the flavor/fragrance composition according to the [3] or [4].
[6] A method for producing a food and/or beverage, an oral composition, a tobacco product, or a consumer product, comprising adding the compound according to the [1] or [2], or the flavor/fragrance composition according to the [3] or [4].

According to the present invention, it is possible to provide a novel compound having a scent useful as a flavor/fragrance ingredient for blending and the like, and a flavor/fragrance composition comprising the compound.

### Description of Embodiments

Hereinafter, the present invention will be described in detail.

As for (5E)-6,10-dimethyl-11-oxoundeca-5,9-dien-2-yl carboxylate of the present invention (hereinafter also referred to as the invention's compound), there are two isomers represented by the following (1a) and (1b) in the case of, for example, acetate esters. Any of these may be used alone or a mixture of these may be used, but the compound (1a) is preferred.

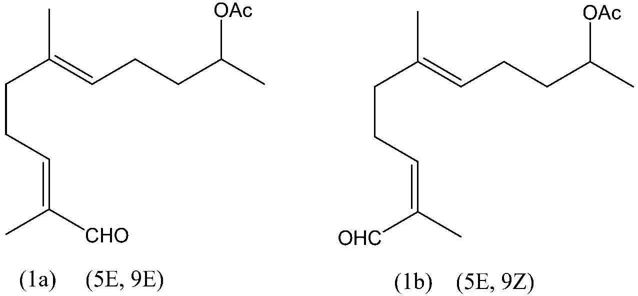

In addition, there are enantiomers represented by the following (1c) and (1d). Any of these may be used alone or a mixture of these may be used.

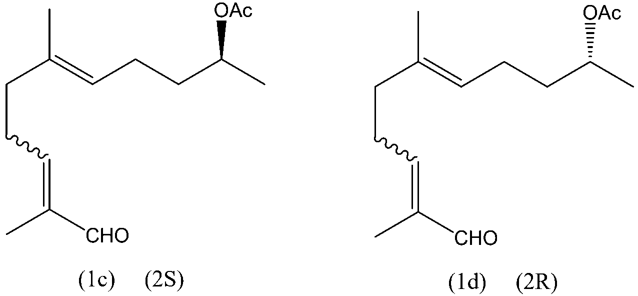

The compounds (1a) and (1b) of the present invention can be prepared in the following steps, for example.

Geranylacetone is reacted with NaBH₄ to reduce the ketone, and then the reaction product is acetylated with acetyl chloride to obtain geranyl acetate. Subsequently, the geranyl acetate is chlorinated with calcium hypochlorite to synthesize allyl chloride, which is hydroxylated via rearrangement with formate and then subjected to a synthesis of allyl alcohol using triethylamine. This is oxidized with manganese dioxide to obtain a mixture of the compounds (1a) and (1b). The above steps are summarized in the following scheme. 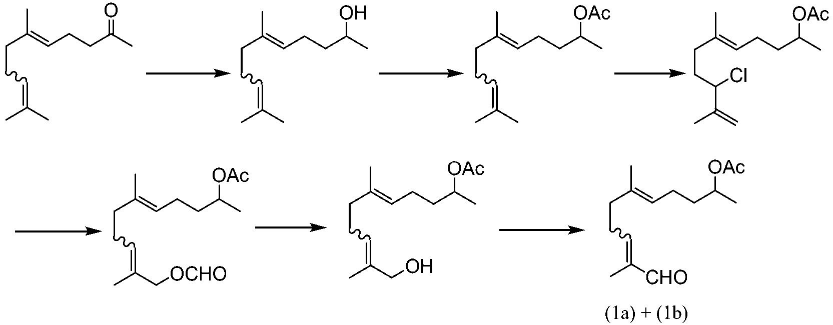 (In the scheme, a wavy line represents cis, trans, or a mixture of cis and trans.)

The compounds (1a) and (1b) of the present invention obtained in this manner can be purified by distillation, various types of chromatography, and so on.

The invention's compound has an excellent scent and may be used alone or in combination with other ingredients as an ingredient for scenting foods and beverages, oral compositions, tobacco products, and so on.

The flavor/fragrance composition of the present invention and the invention's compound can be blended, in extremely small amounts as they are, in foods and beverages to impart or enhance a scent and/or flavor, but they can be also mixed with other ingredients to impart or enhance a scent and/or flavor to foods and beverages. The other flavor/fragrance ingredients that can be mixed include various ingredients such as synthetic flavoring agents, natural flavoring agents, natural essential oils, and plant extracts, and are, for example, natural essential oils, natural flavoring agents, synthetic flavoring agents, and so on listed in "Official Gazette of Japan Patent Office, CUSTOMARY TECHNOLOGY SET (Flavors and Fragrances), PART 2: FOOD FLAVORING, pp. 88-131, published on January 14, 2000".

A concentration range of the invention's compound in the flavor/fragrance composition of the present invention varies depending on other flavor/fragrance ingredients mixed with it and cannot be generalized, but may be adjusted to usually 0.00001 to 10000 ppm, preferably 0.0001 to 1000 ppm, and more preferably 0.001 to 100 ppm based on the weight of the flavor/fragrance composition.

Moreover, in a case where the invention's compound is added to a food or beverage to enhance a scent and/or flavor, a concentration range of the compound can be adjusted to 0.00001 to 10000 ppb, preferably 0.0001 to 1000 ppb, and more preferably 0.001 to 100 ppb based on the weight of the food or beverage.

The flavor/fragrance composition of the present invention containing the invention's compound may contain generally-used ingredients as needed, for example, solvents such as water and ethanol, and fixatives such as ethylene glycol, propylene glycol, dipropylene glycol, glycerol, hexylglycol, benzyl benzoate, triethyl citrate, diethyl phthalate, hercoline, medium-chain fatty acid triglyceride, and medium-chain fatty acid diglyceride.

Adding the flavor/fragrance composition containing the invention's compound or the invention's compound itself makes it possible to enhance scents and/or flavors of foods and/or beverages.

Specific examples of the foods and beverages whose scents and/or flavors can be enhanced by addition of the flavor/fragrance composition of the present invention or the invention's compound include, for example: beverages such as carbonated drinks, soft drinks, fruit juice drinks, fruit liquors, dairy drinks, lactic acid bacteria drinks, energy drinks, soy milk, and tea drinks; desserts such as ice creams, ice milks, lacto ice creams, frozen desserts, yogurts, puddings jellies, and daily desserts; confectioneries such as caramels, candies, tablet candies, crackers, biscuits, cookies, pies, chocolates, snacks, chewing gums, *manju̅*, and *yo̅kan*; soups such as Japanese-style soups, Western-style soups, and Chinese soups; breads; jams; flavor seasonings; various instant drinks; various instant foods; and so on.

The amount of the flavor/fragrance composition added to a food or beverage varies depending on the kind or form of the product, but may be at a concentration range of usually 0.1% and preferably 0.001 to 5% based on the weight of the food or beverage.

Examples of the oral compositions that can be scented and/or flavored by the flavor/fragrance composition of the present invention or the invention's compound include toothpastes, cleaning agents for oral cavity, mouthwashes, troches, chewing gums, and so on. The amount of the flavor/fragrance composition added to an oral composition varies depending on the kind or form of the product, but may be preferably 0.01 to 5% by mass and more preferably 0.1 to 3% by mass relative to the total mass of the oral composition.

Examples of the tobacco products that can be scented and/or flavored by the flavor/fragrance composition of the present invention or the invention's compound include cigarettes, electronic cigarettes, and so on. The amount of the flavor/fragrance composition added to a tobacco product varies depending on the kind or form of the product, but may be, for example, preferably 0.00001 to 90% by mass and more preferably 0.0001 to 50% by mass relative to the total mass of a tobacco leaf portion.

Specific examples of the consumer products include: fragrance products such as perfumes, Eau de Parfum, Eau de Toilette, and Eau de Cologne;
basic cosmetics such as face wash creams, cleansing creams, emulsions, lotions, and cosmetic serums;
makeup cosmetics such as lipsticks, lip balms, eyeliners, mascaras, eye shadows, eyebrow pencils, eye sheets, nail polishes, and polish removers;
hairstyling products such as pomades, brilliantine, setting lotions, hair sticks, hair solids, hair oils, hair treatments, hair creams, hair tonics, liquid hair dressing, hair sprays, bandoline, hair growth products, and hair dyes;
suntanning-related cosmetics such as suntanning products and sunscreen products;
medicated cosmetics such as antiperspirants, aftershave lotions and gels, permanent wave agents, medicated soaps, medicated shampoos, and medicated skin cosmetics;
hair care products such as shampoos, creme rinses, two-in-one shampoos, conditioners, treatments, and hair masks;
soaps such as cosmetic soaps, bath soaps, perfumed soaps, clear soaps, and synthetic soaps;
body cleansers such as body soaps, body shampoos, hand soaps, and face creams;
bath additives (such as bath salts, bath tablets, and bath liquid), foam baths (such as bubble baths), bath oils (such as bath perfumes and bath capsules), and other bath additives such as milk baths, bath jellies, and bath cubes;
detergents such as heavy-duty laundry detergents, light-duty laundry detergents, liquid detergents, laundry soaps, compact detergents, and powdered soaps;
fabric softeners such as softeners and furniture care products;
cleaning agents such as cleansers, house cleaners, toilet cleaners, bathroom cleaners, glass cleaners, mold removers, and drain cleaners;
kitchen detergents such as kitchen soaps, synthetic kitchen soaps, and dishwashing detergents;
bleaching agents such as oxidizing bleaches (such as chlorine bleaches and oxygen bleaches), reducing bleaches (such as sulfur bleaches), and optical bleaching agents;
aerosols such as sprays and powder sprays;
solid-type, gel-type, and liquid-type deodorizers and air fresheners (water-based and oil-based); daily necessities and miscellaneous goods such as tissue paper and toilet paper; quasi-drugs such as liquid bath additives, mouthwashes, and repellents (mist spray type and water-based liquid type); pharmaceuticals such as medicated cosmetics and medicated lotions; and others.

The amount of the invention's compound added to a consumer product may be adjusted at a concentration range of 0.00001 to 10000 ppb, preferably 0.0001 to 1000 ppb, and more preferably 0.001 to 100 ppb relative to the weight of the consumer product.

Furthermore, all the geometric isomers of the invention's compound are useful as scenting ingredients, and any isomer or a mixture of any isomers can be used for flavor/fragrance compositions of the present invention.

### Examples

Hereinafter, the present invention will be described more specifically by using Examples, but the present invention should not be limited to these Examples.

### [Measurement Instruments]

Analytical instruments used in Examples are listed below.
NMR Measurement Equipment: AVANCEIII 500 (manufactured by BRUKER BIO SPIN K.K.)
Gas Chromatograph Mass Spectrometer: GCMS-QP2010 (manufactured by Shimadzu Corporation)
Gas Chromatograph-Orbitrap Mass Spectrometer: TRACE1310 (GC) + Exactive GC (Mass Spectrometer) (manufactured by Thermo Fisher Scientific)
High-Performance Liquid Chromatograph
   Pump: LC-20A (manufactured by Shimadzu Corporation)
   Detector: SPD-M20A (manufactured by GL Sciences)
   Separation Column: YMC-Triart C18 ExRS (manufactured by YMC Corporation)

### (Example 1) Synthesis of Compounds (1a) and (1b)

### (1) Synthesis of (E)-6,10-dimethylundeca-5,9-dien-2-ol

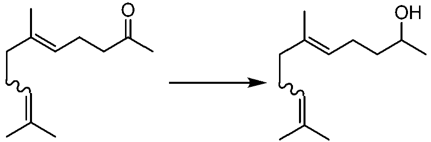

A solution of (E)-6,10-dimethylundeca-5,9-dien-2-one (58.30 g, 0.3 mol) in methanol (250 mL) was cooled to 0°C, sodium borohydride (11.35 g, 0.3 mol) was added to the solution, and the resultant solution was allowed to react for 2 hours. After that, diethyl ether and water were added to the reaction solution and an organic layer was separated. The separated organic layer was purified by distillation to obtain 45.14 g of (E)-6,10-dimethylundeca-5,9-dien-2-ol (yield: 76.6%, GC purity: 92.6%).

### (2) Synthesis of (E)-6,10-dimethylundeca-5,9-dien-2-yl acetate

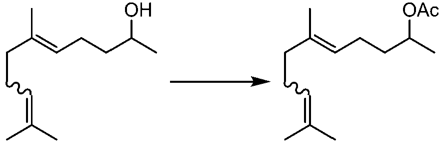

To a solution of (E)-6,10-dimethylundeca-5,9-dien-2-ol (45.14 g, 0.23 mol) obtained in the above (1) in toluene (200 mL), acetyl chloride (19.85 g, 0.25 mol) and pyridine (22.2 mL, 0.28 mol) were added, and the resultant solution was allowed to react at room temperature for 2 hours. Water was added to this reason solution and an organic layer was separated. The separated organic layer was purified by distillation to obtain 49.05 g of (E)-6,10-dimethylundeca-5,9-dien-2-yl acetate (yield: 89.5%, GC purity: 93.1%).

### (3) Synthesis of (E)-9-chloro-6,10-dimethylundeca-5,10-dien-2-yl acetate

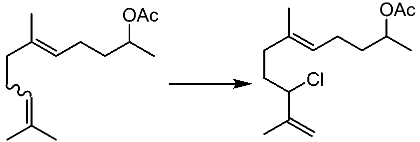

To a solution of (E)-6,10-dimethylundeca-5,9-dien-2-yl acetate (11.92 g, 0.05 mol) obtained in the above (2) in ethyl acetate (120 mL), a solution of calcium hypochlorite (3.57 g, 0.025 mol), tetrabutylammonium bromide (298 mg), and acetic acid (3.30 g, 0.055 mol) in water (15 mL) was added, and the resultant solution was allowed to react at 0°C for 2 hours. Water was added to this reason solution and an organic layer was separated. The separated organic layer was concentrated to obtain 15.47 g of crude (E)-9-chloro-6,10-dimethylundeca-5,10-dien-2-yl acetate.

### (4) Synthesis of (5E)-11-formyloxy-6,10-dimethylundeca-5,9-dien-2-yl acetate

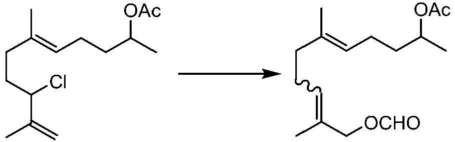

To a solution of a mixture (7.91 g, 0.03 mol) of (E)-9-chloro-6,10-dimethylundeca-5,10-dien-2-yl acetate obtained in the above (3) and 5-chloro-10-methyl-6-methyleneundec-9-en-2-yl acetate in N,N-dimethylformamide (100 mL), tetrabutylammonium iodide (10.71 g, 0.03 mol) and potassium formate (12.20 g, 0.15 mol) were added, and the resultant solution was allowed to react at 80°C for 2 hours. A 5% lithium chloride aqueous solution (200 mL) and hexane were added to this reaction solution, and an organic layer was separated. The separated organic layer was concentrated to obtain crude (5E)-11-formyloxy-6,10-dimethylundeca-5,9-dien-2-yl acetate.

In this synthesis, (Z)-6-formylmethyl-10-methylundeca-5,9-dien-2-yl acetate was produced in 42% as a by-product.

Through purification using silica gel column chromatography (hexane:ethyl acetate = 30:1), 2.93 g of (5E)-11-formyloxy-6,10-dimethylundeca-5,9-dien-2-yl acetate (yield: 39.7%, GC purity: 52.7%) was obtained.

### (5) Synthesis of (5E)-11-hydroxy-6,10-dimethylundeca-5,9-dien-2-yl acetate

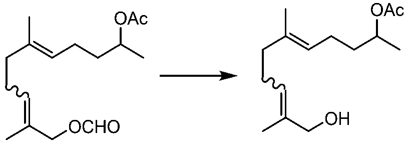

To a solution of (5E)-11-formyloxy-6,10-dimethylundeca-5,9-dien-2-yl acetate (2.93 g, 0.01 mol) obtained in (4) in methanol (50 mL), triethylamine (1.44 mL, 0.01 mol) was added, and the resultant solution was heated under reflux for 2 hours. As a result, 1.56 g of (5E)-11-hydroxy-6,10-dimethylundeca-5,9-dien-2-yl acetate (yield: 59.1%, GC purity: 90.1%) was obtained.

### (6) Synthesis of (5E, 9E)-6,10-dimethyl-11-oxoundeca-5,9-dien-2-yl acetate and (5E, 9Z)-6,10-dimethyl-11-oxoundeca-5,9-dien-2-yl acetate

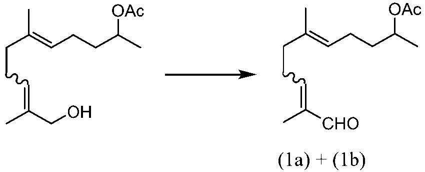

To a solution of (5E)-11-hydroxy-6,10-dimethylundeca-5,9-dien-2-yl acetate (1.56 g, 0.006 mol) obtained in (5) in hexane (50 mL), manganese dioxide (5.33 g, 0.06 mol) was added and the resultant solution was allowed to react at room temperature for 8 hours. The reaction solution thus obtained was purified by using silica gel column chromatography (hexane:ethyl acetate = 30:1) to obtain 0.6 g of a mixture of (5E, 9E)-6,10-dimethyl-11-oxoundeca-5,9-dien-2-yl acetate (1a) and (5E, 9Z)-6,10-dimethyl-11-oxoundeca-5,9-dien-2-yl acetate (1b) (yield: 38.7%, GC purity: 90.4%, 9Z/9E = 68/32).

The synthesized mixture was purified with HPLC (eluent; water:acetonitrile = 36:64 (volume ratio)) to obtain (5E, 9E)-6,10-dimethyl-11-oxoundeca-5,9-dien-2-yl acetate (1a) (purity 100%) and (5E, 9Z)-6,10-dimethyl-11-oxoundeca-5,9-dien-2-yl acetate (1b) (purity 100%).

### <Physical data of (5E, 9E)-6,10-dimethyl-11-oxoundeca-5,9-dien-2-yl acetate>

1H NMR (500MHz, CDCl3) δ:
9.39 (s, 1H), 6.46 (t, J = 7.33Hz, 1H), 5.15 (t, J = 7.16Hz, 1H), 4.88 (m, 1H), 2.46 (q, J = 7.33Hz, 2H), 2.17 (t, J = 7.33Hz, 2H), 2.03 (s, 3H), 2.03 (m, 2H), 1.48-1.68 (m, 2H), 1.75 (s, 3H), 1.62 (s, 3H), 1.21 (d, J = 6.25Hz, 3H)
13C NMR (125MHz, CDCl3) δ:
   195.31, 170.72, 154.26, 139.42, 134.20, 124.71, 70.46, 37.96, 35.80, 27.32, 23.88, 21.36, 19.97, 15.80, 9.21
   HRMS(CI+): calcd C15H25O3([M+H]+)253.1798; found, 253.1799

### <Physical data of (5E, 9Z)-6,10-dimethyl-11-oxoundeca-5,9-dien-2-yl acetate>

1H NMR (500MHz,CDCl3) δ:
10.12 (s, 1H), 6.48 (t, J = 7.51Hz, 1H), 5.13 (t, J = 7.15Hz, 1H), 4.88 (m, 1H), 2.66 (q, J = 7.51Hz, 2H), 2.15 (t, J = 7.51Hz, 2H), 2.03 (s, 3H), 2.03 (m, 2H), 1.48-1.68 (m, 2H), 1.77 (s, 3H), 1.60 (s, 3H), 1.21 (d, J = 6.25Hz, 3H)
13C NMR (125MHz,CDCl3) δ:
   191.13, 170.72, 149.00, 136.16, 133.90, 125.07, 70.47, 39.32, 35.77, 25.08, 23.87, 21.35, 19.96, 15.85, 9.20
   HRMS(CI+): calcd C15H25O3([M+H]+)253.1798; found, 253.1797

### (Example 2) Flavor/Fragrance Composition for Grapefruit Foods

A flavor/fragrance composition for grapefruit foods (A) was prepared in accordance with the following recipe using the invention's compound produced in the method described in Example 1. Mixture in Example 1 refers to the mixture of (5E, 9E)-6,10-dimethyl-11-oxoundeca-5,9-dien-2-yl acetate and (5E, 9Z)-6,10-dimethyl-11-oxoundeca-5,9-dien-2-yl acetate (the same applies to the following Examples).

### <Recipe of Flavor/Fragrance Composition (A)>

| (Ingredient) | (parts by weight) |
|---|---|
| Mixture in Example 1 | 0.0001 |
| Grapefruit Oil (Cold Pressed) | 50.0 |
| Ethanol | Balance |
| Total | 1000.0 |

### (Comparative Example 1)

As Comparative Example 1, a flavor/fragrance composition for grapefruit foods (B) was prepared according to the following recipe.

### <Recipe of Flavor/Fragrance Composition (B)>

| (Ingredient) | (parts by weight) |
|---|---|
| Grapefruit Oil (Cold Pressed) | 50.0 |
| Ethanol | Balance |
| Total | 1000.0 |

### (Comparison Experiment Example 1)

The flavor/fragrance compositions for grapefruit foods (A) and (B) obtained in Example 2 and Comparative Example 1 were each added to water at 0.1%. A sensory test was conducted by six professional panelists. All the panelists pointed out that the flavor/fragrance composition (A) in Example 2 imparted a citrus-like juicy voluminous flavor and a juice-vesicle-like popping texture and therefore was significantly superior to the flavor/fragrance composition (B) in Comparative Example 1. The same result was obtained when the 5E, 9E isomer or the 5E, 9Z isomer was used alone.

### (Example 3) Addition to Commercially Available Grapefruit Juice Drink

The 5E, 9E isomer or the 5E, 9Z isomer obtained in Example 1 or the mixture of them was added at 0.1 ppb to a commercially available grapefruit juice drink and a sensory test was conducted by six professional panelists. All the panelists pointed out that the addition of the 5E, 9E isomer, the 5E, 9Z isomer, or the mixture of them imparted the drink a rich voluminous juicy flavor and a natural fruity flavor, making the drink significantly superior.

### (Example 4) Flavor/Fragrance Composition for Grapefruit Foods

A flavor/fragrance composition for grapefruit foods (C) was prepared according to the following recipe.

### <Recipe of Flavor/Fragrance Composition (C)>

| (Ingredient) | (parts by weight) |
|---|---|
| Mixture in Example 1 | 0.0001 |
| Nootkatone | 2.0 |
| Octanal | 0.3 |
| Decanal | 0.2 |
| Dodecanal | 0.1 |
| Linalool | 0.8 |
| Cis-3-Hexenoll | 0.5 |
| Ethanol | Balance |
| Total | 1000.0 |

### (Comparative Example 2)

As Comparative Example 2, a flavor/fragrance composition for grapefruit foods (D) was prepared according to the following recipe.

### <Recipe of Flavor/Fragrance Composition (D)>

| (Ingredient) | (parts by weight) |
|---|---|
| Nootkatone | 2.0 |
| Octanal | 0.3 |
| Decanal | 0.2 |
| Dodecanal | 0.1 |
| Linalool | 0.8 |
| Cis-3-Hexenoll | 0.5 |
| Ethanol | Balance |
| Total | 1000.0 |

### (Comparison Experiment Example 2)

The flavor/fragrance compositions for grapefruit foods (C) and (D) obtained in Example 4 and Comparative Example 2 were each added to water at 0.1%. A sensory test was conducted by six professional panelists. All the panelists pointed out that the flavor/fragrance composition (C) in Example 4 imparted a citrus-like juicy voluminous flavor and a juice-vesicle-like popping texture and therefore was significantly superior to the flavor/fragrance composition (D) in Comparative Example 2. The same result was obtained when the 5E, 9E isomer or the 5E, 9Z isomer was used alone.

### (Example 5) Flavor/Fragrance Composition for Orange Foods

A flavor/fragrance composition for orange foods (E) was prepared according to the following recipe.

### <Recipe of Flavor/Fragrance Composition (E)>

| (Ingredient) | (parts by weight) |
|---|---|
| Mixture in Example 1 | 0.0001 |
| Orange Oil (Cold Pressed) | 50.0 |
| Ethanol | Balance |
| Total | 1000.0 |

### (Comparative Example 3)

As Comparative Example 3, a flavor/fragrance composition for orange foods (F) was prepared according to the following recipe.

### <Recipe of Flavor/Fragrance Composition (F)>

| (Ingredient) | (parts by weight) |
|---|---|
| Orange Oil (Cold Pressed) | 50.0 |
| Ethanol | Balance |
| Total | 1000.0 |

### (Comparison Experiment Example 3)

The flavor/fragrance compositions for orange foods (E) and (F) obtained in Example 5 and Comparative Example 3 were each added to water at 0.1%. A sensory test was conducted by six professional panelists. All the panelists pointed out that the flavor/fragrance composition (E) in Example 5 enhanced a natural peel flavor and a citrus-like voluminous flavor and therefore was significantly superior to the flavor/fragrance composition (F) in Comparative Example 3. The same result was obtained when the 5E, 9E isomer or the 5E, 9Z isomer was used alone.

### (Example 6) Addition to Commercially Available Orange Juice Drink

The 5E, 9E isomer or the 5E, 9Z isomer obtained in Example 1 or the mixture of them was added at 0.1 ppb to a commercially available orange juice drink and a sensory test was conducted by six professional panelists. All the panelists pointed out that the addition of the 5E, 9E isomer, the 5E, 9Z isomer, or the mixture of them enhanced a natural peel flavor and a fruity flavor, making the drink significantly superior.

### (Example 7) Flavor/Fragrance Composition for Orange Foods

A flavor/fragrance composition for orange foods (G) was prepared according to the following recipe.

### <Recipe of Flavor/Fragrance Composition (G)>

| (Ingredient) | (parts by weight) |
|---|---|
| Mixture in Example 1 | 0.0001 |
| Ethyl acetate | 0.5 |
| Octanal | 1.0 |
| Nonanal | 0.1 |
| Decanal | 0.5 |
| Linalool | 2.0 |
| Ethanol | Balance |
| Total | 1000.0 |

### (Comparative Example 4)

As Comparative Example 4, a flavor/fragrance composition for orange foods (H) was prepared according to the following recipe.

### <Recipe of Flavor/Fragrance Composition (H)>

| (Ingredient) | (parts by weight) |
|---|---|
| Ethyl acetate | 0.5 |
| Octanal | 1.0 |
| Nonanal | 0.1 |
| Decanal | 0.5 |
| Linalool | 2.0 |
| Ethanol | Balance |
| Total | 1000.0 |

### (Comparison Experiment Example 4)

The flavor/fragrance compositions for orange foods (G) and (H) obtained in Example 7 and Comparative Example 4 were each added to water at 0.1%. A sensory test was conducted by six professional panelists. All the panelists pointed out that the flavor/fragrance composition (E) in Example 5 imparted a natural peel flavor and a fruity flavor and therefore was significantly superior to the flavor/fragrance composition (H) in Comparative Example 4. The same result was obtained when the 5E, 9E isomer or the 5E, 9Z isomer was used alone.

### (Example 8) Flavor/Fragrance Composition for Lemon Foods

A flavor/fragrance composition for lemon foods (I) was prepared according to the following recipe.

### <Recipe of Flavor/Fragrance Composition (I)>

| (Ingredient) | (parts by weight) |
|---|---|
| Mixture in Example 1 | 0.0001 |
| Lemon Oil (Cold Pressed) | 50.0 |
| Ethanol | Balance |
| Total | 1000.0 |

### (Comparative Example 5)

As Comparative Example 5, a flavor/fragrance composition for lemon foods (J) was prepared according to the following recipe.

### <Recipe of Flavor/Fragrance Composition (J)>

| (Ingredient) | (parts by weight) |
|---|---|
| Lemon Oil (Cold Pressed) | 50.0 |
| Ethanol | Balance |
| Total | 1000.0 |

### (Comparison Experiment Example 5)

The flavor/fragrance compositions for lemon foods (I) and (J) obtained in Example 8 and Comparative Example 5 were each added to water at 0.1%. A sensory test was conducted by six professional panelists. All the panelists pointed out that the flavor/fragrance composition (I) in Example 8 enhanced a natural peel flavor and a citrus-like voluminous flavor and therefore was significantly superior to the flavor/fragrance composition (J) in Comparative Example 5. The same result was obtained when the 5E, 9E isomer or the 5E, 9Z isomer was used alone.

### (Example 9) Addition to Commercially Available Lemon Juice Drink

The 5E, 9E isomer or the 5E, 9Z isomer obtained in Example 1 or the mixture of them was added at 0.1 ppb to a commercially available lemon juice drink and a sensory test was conducted by six professional panelists. All the panelists pointed out that the addition of the 5E, 9E isomer, the 5E, 9Z isomer or the mixture of them enhanced a natural peel flavor and a fruity flavor, making the drink significantly superior.

### (Example 10) Flavor/Fragrance Composition for Lemon Foods

A flavor/fragrance composition for lemon foods (K) was prepared according to the following recipe.

### <Recipe of Flavor/Fragrance Composition (K)>

| (Ingredient) | (parts by weight) |
|---|---|
| Mixture in Example 1 | 0.0001 |
| Citral | 3.0 |
| α-Terpineol | 1.0 |
| Geraniol | 0.5 |
| Geranyl Acetate | 0.5 |
| Neryl Acetate | 0.5 |
| Ethanol | Balance |
| Total | 1000.0 |

### (Comparative Example 6)

As Comparative Example 6, a flavor/fragrance composition for lemon foods (L) was prepared according to the following recipe.

### <Recipe of Flavor/Fragrance Composition (L)>

| (Ingredient) | (parts by weight) |
|---|---|
| Citral | 3.0 |
| α-Terpineol | 1.0 |
| Geraniol | 0.5 |
| Geranyl Acetate | 0.5 |
| Neryl Acetate | 0.5 |
| Ethanol | Balance |
| Total | 1000.0 |

### (Comparison Experiment Example 6)

The flavor/fragrance compositions for lemon foods (K) and (L) obtained in Example 10 and Comparative Example 6 were each added to water at 0.1%. A sensory test was conducted by six professional panelists. All the panelists pointed out that the flavor/fragrance composition (K) in Example 10 imparted a natural peel flavor and a fruity flavor and therefore was significantly superior to the flavor/fragrance composition (L) in Comparative Example 6. The same result was obtained when the 5E, 9E isomer or the 5E, 9Z isomer was used alone.

## Claims

1. A compound represented by the following formula (1): 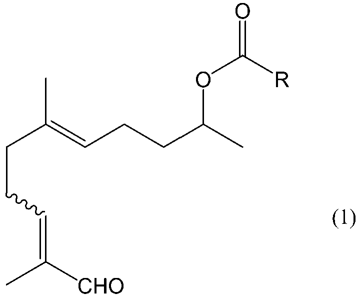 where R represents an alkyl group having 1 to 4 carbon atoms and a wavy line represents cis, trans, or a mixture of cis and trans.

2. The compound according to claim 1, wherein R is a methyl group.

3. A flavor/fragrance composition comprising the compound according to claim 1 or 2.

4. The flavor/fragrance composition according to claim 3, which is for a food and/or beverage.

5. A food and/or beverage, an oral composition, a tobacco product, or a consumer product comprising the compound according to claim 1 or 2, or the flavor/fragrance composition according to claim 3 or 4.

6. A method for producing a food and/or beverage, an oral composition, a tobacco product, or a consumer product, comprising adding the compound according to claim 1 or 2, or the flavor/fragrance composition according to claim 3 or 4.
